# FASCICULE DE BREVET EUROPEEN

(11) **EP 1 212 104 B1**
(45) Date de publication et mention de la délivrance du brevet: **16.06.2004**
(21) Numéro de dépôt: 00962628.4
(22) Date de dépôt: 13.09.2000
(51) Int. Cl.: A61L 9/03, G05B 19/00

(54) **DISPOSITIF DYNAMIQUE DE DIFFUSION DE PARFUMS ET PROCEDE DE PILOTAGE DE CE DISPOSITIF**
DYNAMISCHER DUFTSTOFFSPENDER UND STEUERUNGSVERFAHREN DIESES SPENDERS
DYNAMIC DEVICE FOR PERFUME DIFFUSION AND METHOD FOR CONTROLLING SAME

(30) Priorité: 14.09.1999 FR 9911485
(43) Date de publication de la demande: 12.06.2002
(73) Titulaire: FRANCE TELECOM, 75015 Paris (FR)
(72) Inventeur: BALBI, Dominique, F-92270 Bois-Colombes (FR); MESSAGER, Jacques, F-35340 Liffre (FR)
(74) Mandataire: Jacobson, Claude
(86) Numéro de dépôt international: PCT/FR2000/002531
(87) Numéro de publication internationale: WO 2001/019417

(56) Documents cités:
- EP-A- 0 831 384
- WO-A-97/37693
- US-A- 4 629 604
- US-A- 5 398 070

## Description

La présente invention est relative à un dispositif de diffusion de parfums, particulièrement adapté pour être associé à un appareil électronique, tel qu'un appareil électronique grand public, notamment un micro-ordinateur, un poste de télévision, un décodeur de télévision numérique, un magnétoscope, un terminal multimédia ou analogue.

Elle se rapporte également à un procédé de diffusion de parfums mis en oeuvre au moyen d'un tel appareil.

Le but de l'invention est de permettre la diffusion de matières odorantes provenant d'échantillons de parfums en synchronisme avec des informations, notamment des images, des sons, des mots, ou analogues présentées à un utilisateur sur ou par l'appareil électronique.

Le document WO-A-97 37693 décrit un dispositif de diffusion de parfums incorporé dans un appareil de télévision et dont le fonctionnement est initialisé et/ou stoppé par un signal provenant par exemple d'un équipement extérieur. Un tel dispositif intègre de manière implicite un algorithme de pilotage de son fonctionnement.

Le document US-A-5 398 070 décrit également un appareil de télévision comprenant un dispositif de diffusion de parfums et adapté pour recevoir un signal comprenant simultanément des informations d'images, de sons et d'odeurs. Ce dispositif comprend notamment un micro-processeur chargé du pilotage de la restitution d'images, de sons et d'odeurs.

L'invention à pour objet un dispositif de diffusion de parfums en synchronisme avec des informations, notamment des images, des sons, des mots ou analogues, présentées à un utilisateur, tel que défini dans la revendication 1.

Ce dispositif de diffusion de parfums peut également comporter une ou plusieurs des caractéristiques des revendications dépendantes suivantes, prises isolément ou selon toutes les combinaisons techniquement possibles.

Un autre objet de l'invention est un procédé de diffusion de parfums, tel que défini dans la revendication 5, en synchronisme avec des informations, notamment des images, des sons, des mots ou analogues, présentées à un utilisateur au moyen d'un appareil doté d'un dispositif tel que défini précédemment.

Ce procédé de diffusion de parfums peut également comporter une ou plusieurs des étapes des revendications dépendantes suivantes, prises isolément ou selon toutes les combinaisons techniquement possibles.

D'autres caractéristiques et avantages ressortiront de la description suivante, donnée uniquement à titre d'exemple, et faite en référence aux dessins annexés sur lesquels :
- la Fig. 1 est une vue schématique en perspective d'un dispositif de diffusion de parfums conforme à l'invention,
- la Fig. 2 est un schéma synoptique montrant un exemple de réalisation du dispositif de diffusion de la Fig. 1 ;
- la Fig. 3 est une vue agrandie des moyens de stockage d'échantillons de parfum incorporés au dispositif de la Fig. 1 ; et
- la Fig. 4 est un organigramme montrant les différentes phases du procédé mis en oeuvre au moyen du dispositif de la Fig. 1.

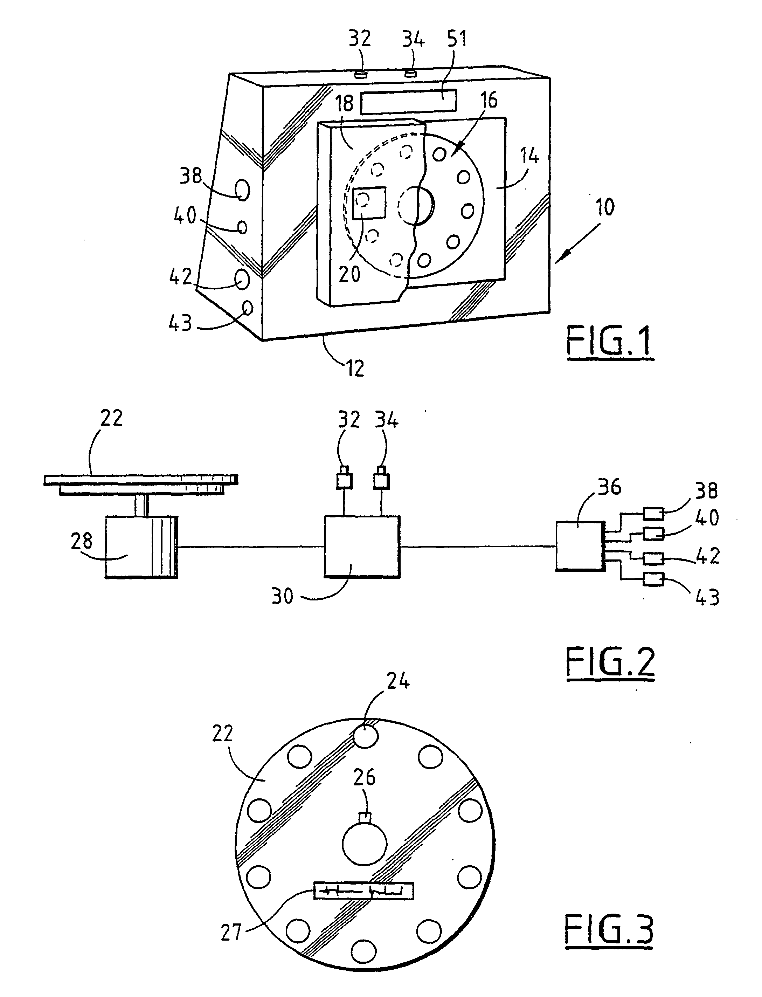

Sur la Fig. 1, on a représenté un dispositif de diffusion de parfums conforme à l'invention, désigné par la référence numérique générale 10.

Il est destiné à provoquer l'émission de matières odorantes à partir d'échantillons de parfum ou de senteur stockés dans des moyens de stockage d'échantillons appropriés, et ce, en synchronisme avec des informations présentées à un utilisateur, en particulier des images, des sons, des mots ou analogues.

Comme on le conçoit, il est destiné à être associé à un appareil électronique grand public, tel qu'un micro-ordinateur, un terminal multimédia, un poste de télévision numérique, un décodeur de télévision numérique ou un magnétoscope, sur lequel ou au moyen duquel les informations sont présentées à l'utilisateur. Il permet donc l'émission de parfums correspondant, par exemple, à des images affichées sur un écran.

Comme on le voit sur la Fig. 1, le dispositif 10 comporte principalement un boîtier 12 muni d'un réceptacle 14 dans lequel sont disposés les moyens de stockage d'échantillons 16 amovibles, et de moyens d'alimentation en tension (non représentés).

Le réceptacle 14 est obturé par un volet articulé 18 permettant le chargement et le déchargement du réceptacle 14, ce volet 18 étant muni d'une fenêtre 20 à travers laquelle les échantillons de parfum peuvent être mis en communication avec l'air ambiant.

En se référant également aux Figs. 2 et 3, les moyens de stockage des échantillons de parfum sont constitués par un disque 22 supportant un ensemble de réservoirs de stockage des échantillons de parfum, tels que 24, dont l'un au moins est vide ou empli d'une matière non odorante ou neutralisante.

Le disque et les récipients sont chacun identifiables par une adresse.

Le récipient vide est destiné à être positionné en regard de la fenêtre de diffusion 20 lorsque le dispositif se situe en position de repos.

Un détrompeur 26 permet le positionnement angulaire précis du disque 22 dans le réceptacle 14 et une fenêtre 27 permet à l'utilisateur d'identifier le disque.

Le disque 22 est associé à des moyens moteurs 28 assurant la mise en contact sélective des échantillons avec l'air ambiant, c'est à dire qu'ils positionnent l'un des réservoirs 24 en regard de la fenêtre de diffusion 20, et ce en fonction des informations qui sont présentées à l'utilisateur, de manière à diffuser un parfum correspondant, par exemple à des images qui lui sont présentées.

Ces moyens moteurs 28 sont constitués par un moteur pas à pas commandé par des moyens de commande 30.

Ces moyens de commande sont constitués par une unité centrale dans laquelle sont stockés des ordres ou un algorithme de pilotage du moteur 28.

L'unité centrale est raccordée à des boutons-poussoir 32 et 34, constituant des moyens de commande secondaire actionnables manuellement par l'utilisateur pour provoquer la rotation du disque 22 de manière à positionner l'un des réservoirs 24 en regard de la fenêtre de diffusion 20.

On peut ainsi, à volonté, diffuser des matières odorantes même en l'absence d'informations visuelles ou sonores correspondantes.

On voit par ailleurs sur la Fig. 2 que l'unité centrale 30 est raccordée à un circuit d'interface 36, de type classique, elle-même connectée à des prises 38, 40, 42 et 43 pour le raccordement du dispositif 10 à un équipement électronique.

Comme cela a été mentionné précédemment, cet équipement peut être constitué par un micro-ordinateur, un terminal multimédia, un poste de télévision numérique, un décodeur de télévision numérique ou un magnétoscope.

Bien entendu, il peut être associé à tout type de dispositif électronique capable de présenter des informations visuelles ou sonores.

Dans le cas ou le dispositif 10 est raccordé à un magnétoscope, l'algorithme de pilotage du moteur pas à pas 28 est intégré au micro-contrôleur du magnétoscope, lors de la fabrication de celui-ci.

Lorsqu'il est raccordé à un poste de télévision numérique ou un décodeur de télévision numérique, l'algorithme de pilotage se présente sous la forme d'un logiciel téléchargé, également connu sous l'application "appliquette-java", ce logiciel étant transmis par l'émetteur des signaux de télévision dans le flux d'informations transmises à l'utilisateur en utilisant un canal spécifique associé à la signalisation DVB ("Digital Video Broadcasting") puis stocké dans le dispositif.

Enfin, lorsqu'il est associé à un micro-ordinateur, l'algorithme de pilotage se présente également sous la forme d'un programme téléchargé ou "appliquette-java", ce programme étant chargé lorsque le micro-ordinateur communique la première fois avec un centre serveur approprié pour fournir un tel service, puis stocké dans le dispositif.

Dans ce cas, l'une des pages hypertexte récupérée sur le centre serveur, mise en page selon le format HTML (Hypertext Mark-Up Language) présente un lien actif pour récupérer le logiciel stocké dans une zone mémoire correspondante, par exemple dans le même centre serveur, ou dans un centre serveur distinct, ce logiciel de pilotage étant transmis vers le micro-ordinateur puis vers le dispositif de diffusion 10.

On notera que dans les différents modes de réalisation qui viennent d'être envisagés, le dispositif 10 est raccordé, de préférence, aux bornes de raccordement à la carte son de l'appareil électronique ou sur le bus USB du micro-ordinateur.

Ainsi le logiciel de pilotage du moteur 28 subit un traitement identique à des fichiers sons transmis classiquement à des enceintes acoustiques équipant l'appareil pour diffuser des signaux sonores.

On notera également, que dans le cas d'un micro-ordinateur, l'algorithme de pilotage peut également être fourni et/ou activé sous la forme d'un logiciel stocké sur un disque de type "CD-Rom".

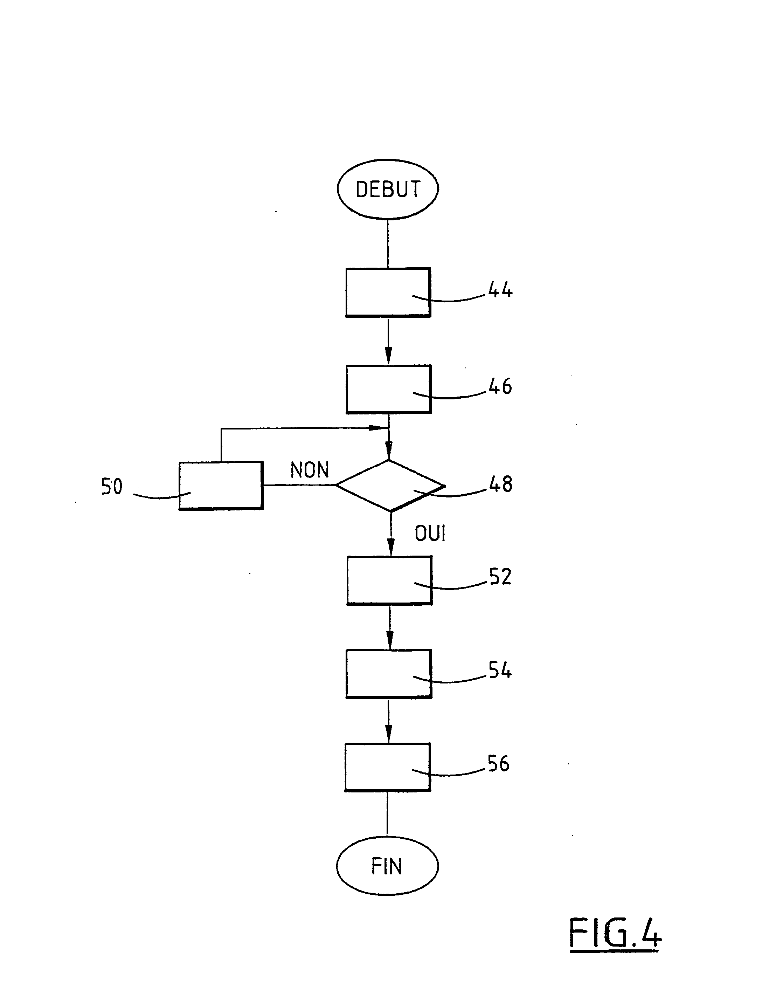

Le procédé de fonctionnement du dispositif qui vient d'être décrit va maintenant être exposé en référence à la Fig. 4.

Cette procédure débute par une première étape 44 au cours de laquelle le dispositif réceptionne l'algorithme de pilotage du moteur 28, comme cela a été mentionné précédemment (téléchargement de l'appliquette).

Elle se poursuit ensuite par différentes étapes d'exécution de cet algorithme.

Pour ce faire, au cours d'une première étape 46, le dispositif 10 est activé. En réponse, celui-ci transmet un accusé de réception à l'émetteur de l'algorithme de pilotage.

On fournit ensuite l'identifiant du disque 22 à insérer dans le réceptacle 14 et après acquittement par l'utilisateur par la fermeture du volet 18, l'algorithme de pilotage est activé (étape 48).

Si tel n'est pas le cas, c'est à dire si l'utilisateur n'a pas inséré le disque, la procédure se poursuit par une étape 50 au cours de laquelle on invite à nouveau l'utilisateur à insérer le disque approprié en utilisant un afficheur 51 (figure 1) prévu à cet effet. Cet afficheur 51 est également utilisé, de façon générale, pour l'affichage d'informations concernant les senteurs dégagées.

Lors de l'étape 52 suivante, dans le cas ou le disque a été inséré dans le réceptacle 14, l'unité centrale 30 du dispositif 10 transmet au moteur 28 l'adresse du réservoir qu'il convient de positionner en regard de la fenêtre 20 de diffusion.

Lors de l'étape 54 suivante, un ordre de diffusion est émis vers les moyens moteurs de manière à provoquer la rotation du disque jusqu'à une position angulaire appropriée.

De façon optionnelle, au cours de cette étape, une durée de diffusion peut également être transmise aux moyens moteurs.

De même, au cours de cette étape, simultanément à la transmission de l'ordre de diffusion, un message destiné à l'afficheur 51 peut être transmis pour fournir des informations relatives à la senteur contenue dans le réservoir adressé.

A l'issu de cette étape 54, ou lorsque la durée de diffusion prévue est écoulée, l'unité centrale 30 transmet au moteur 28 un ordre d'arrêt de diffusion. En réponse, le moteur 28 provoque la rotation du disque 22 de manière à positionner le réceptacle 24 qui est dépourvu de matière odorante en regard de la fenêtre 20.

En variante, il est possible de provoquer la rotation du disque 22 de manière à positionner, en regard de la fenêtre 20, un réservoir emplit d'une matière neutralisante appropriée capable d'absorber les odeurs.

Cette variante est avantageuse dans le cas ou l'on souhaite provoquer l'émission d'un autre parfum.

Si tel est le cas, l'unité centrale 30 retourne à l'étape 52 précédente de manière à transmettre au moteur 28 une nouvelle adresse correspondant à un autre réservoir, ainsi qu'un autre ordre de diffusion.

Lorsque la séquence de diffusion est achevée, la procédure se poursuit par une étape 56 au cours de laquelle le dispositif reçoit un ordre de désactivation.

Dans le cas du couplage du dispositif à un micro-ordinateur, cet ordre de désactivation peut s'effectuer de façon automatique dès que la page HTML contenant le lien adressant la zone mémoire contenant l'algorithme de pilotage est abandonnée.

La procédure qui vient d'être décrite peut également être remplacée par un mode de fonctionnement manuel, selon lequel l'utilisateur choisit lui-même, à volonté, les parfums qu'il souhaite diffuser, en actionnant les boutons-poussoirs 32 et 34.

On notera que l'invention n'est pas limitée au mode de réalisation décrit.

En effet, dans la description qui vient d'être faite, les moyens de stockage des échantillons de parfum sont constitués par un disque rotatif associé à des moyens moteurs de commande de leur position angulaire.

Il est également possible, en variante, de doter le dispositif de moyens de stockage d'échantillons de parfum se présentant sous la forme d'une carte dotée d'alvéoles frangibles emplies chacune d'un échantillon de parfum.

Dans ce cas, les moyens de commande de la mise en contact sélective des échantillons avec l'air ambiant sont par exemple constitués par des électrodes assurant la fusion sélective de la paroi des alvéoles.

Il est également possible de doter le dispositif, dans les deux modes de réalisation envisagés, de moyens de ventilation ou de chauffage, permettant d'améliorer la diffusion des matières odorantes.

On conçoit que l'invention qui vient d'être décrite, qui permet d'associer des informations, telles que des informations visuelles, ou sonores, à des odeurs permet, par exemple, de diffuser des senteurs marines lorsqu'un paysage marin est présenté sur un écran.

Elle permet ainsi, par exemple, à des industriels du domaine de la parfumerie ou de la cosmétique d'associer à un site accessible par réseau informatique, par exemple un site Internet, des senteurs permettant de présenter au public des échantillons de parfum proposés à la vente, mais aussi de créer de nouvelles senteurs associées à des atmosphères régnant dans une scène correspondant à des images affichées, par exemple dans le cadre de jeux éducatifs.

## Revendications

1. Dispositif de diffusion de parfums en synchronisme avec des informations, notamment des images, des sons, des mots ou analogues, présentées à un utilisateur, comportant un réceptacle (14) dans lequel sont placés des moyens (16, 22) de stockage d'échantillons de parfum et qui est doté de moyens (28) de mise en contact sélective d'un échantillon avec l'air ambiant, sous la commande de moyens (30) de pilotage comportant des moyens de stockage d'un algorithme de pilotage du fonctionnement desdits moyens (28) de mise en contact d'un échantillon avec l'air ambiant en fonction des informations présentées à l'utilisateur, **caractérisé en ce que** lesdits moyens de stockage sont adaptés pour recevoir par téléchargement ledit algorithme à partir d'un équipement électronique et le stocker en vue de son exécution.

2. Dispositif selon la revendication 1, **caractérisé en ce que** les moyens (16, 22) de stockage d'échantillons comprennent un disque rotatif (22) supportant un ensemble de réservoirs (24) de stockage des échantillons de parfum et **en ce que** les moyens de mise en contact d'un échantillon avec l'air ambiant comportent des moyens (28) de moteur de commande du déplacement angulaire du disque pour le positionnement de l'un des réservoirs en regard d'une fenêtre (20) de diffusion de parfums, en fonction de l'algorithme de pilotage.

3. Dispositif selon l'une des revendications 1 ou 2, **caractérisé en ce que** les moyens (16, 22) de stockage d'échantillons comprennent une carte comportant des alvéoles frangibles emplies de parfum.

4. Dispositif selon l'une des revendications 1 à 3, caractérisé en qu'il est doté d'au moins une prise (38, 40, 42, 43) pour son raccordement sur une sortie dudit équipement électronique choisi parmi un ordinateur, un terminal multimédia, un poste de télévision numérique, un décodeur de télévision numérique et un magnétoscope.

5. Procédé de diffusion de parfums en synchronisme avec des informations, notamment des images, des sons, des mots ou analogues, présentées à un utilisateur, au moyen d'un appareil électronique doté d'un dispositif selon l'une quelconque des revendications 1 à 4, **caractérisé en ce qu'**il consiste à, simultanément à la présentation à l'utilisateur d'une séquence d'informations prédéterminée, télécharger vers les moyens de stockage des moyens (30) de pilotage du dispositif (10) de diffusion de parfums, un algorithme de pilotage des moyens de mise en contact sélective d'un échantillon de parfum avec l'air ambiant en fonction d'informations présentées à l'utilisateur, pour le stocker en vue d'une étape d'exécution.

6. Procédé selon la revendication 5, **caractérisé en ce que** l'étape d'exécution de l'algorithme de pilotage comporte les étapes suivantes:
- activation du dispositif (10) de diffusion de parfums ;
- émission d'un message d'identification des moyens (16) de stockage d'échantillons disposés dans le réceptacle ;
- adressage d'un réservoir (24) ou d'une alvéole empli d'un échantillon de parfum;
- diffusion de l'échantillon de parfum contenu dans le réservoir (24) ou l'alvéole adressé ;
- après une période de temps prédéterminée, arrêt de diffusion de parfum ; et
- désactivation du dispositif (10).

7. Procédé selon la revendication 6, **caractérisé en ce que**, postérieurement à l'étape d'arrêt de diffusion, il comporte au moins une deuxième étape d'adressage d'un autre réservoir (24) ou alvéole et au moins une deuxième étape de diffusion de l'échantillon de parfum correspondant.

8. Procédé selon l'une des revendications 5 à 7, **caractérisé en ce que** l'appareil électronique est constitué par un micro-ordinateur ou un terminal multimédia dans lequel est chargé un algorithme de navigation sur un réseau informatique, pour l'accès à des centres serveurs, par l'intermédiaire d'un fournisseur d'accès, et **en ce que** l'algorithme de pilotage est téléchargé à partir d'un lien, contenu dans une page hypertexte stockée dans un centre serveur avec lequel communique le micro-ordinateur, ledit lien provoquant le téléchargement de l'algorithme de pilotage, à partir d'une zone mémoire dans laquelle il est stocké, vers le micro-ordinateur.

9. Procédé selon l'une des revendications 5 à 8, **caractérisé en ce que** l'appareil électronique est constitué par un décodeur de télévision numérique, l'algorithme de pilotage étant transmis et/ou activé par l'émetteur des signaux de télévision numérique puis stocké dans les moyens de pilotage (30) du dispositif (10).

## Patentansprüche

1. Vorrichtung zur Diffusion von Duftstoffen synchron mit Informationen, insbesondere Bildern, Tönen, Worten oder Ähnlichem, die einem Benutzer dargeboten werden, welche einen Aufnahmeraum (14) aufweist, in dem Vorrichtungen (16, 22) zur Lagerung von Duftstoffproben vorgesehen sind, und in dem sich Mittel (28) befinden, die geeignet sind, eine Probe selektiv in Kontakt mit der Umgebungsluft zu bringen und die von einer Steuereinrichtung (30) angesteuert sind, welche einen Speicher für einen Algorithmus zum Steuern der Funktionsweise dieser Mittel (28) aufweisen,
**dadurch gekennzeichnet,**
**dass** dieser Speicher geeignet ist, um den Algorithmus durch Hochladen von einer elektronischen Einrichtung zu erhalten und im Hinblick auf seine Ausführung zu speichern.

2. Vorrichtung nach Anspruch 1,
**dadurch gekennzeichnet,**
**dass** die Vorrichtungen (16, 22) zur Lagerung von Duftstoffproben eine Drehscheibe (22) aufweisen, die einen Satz von Behältern (24) zur Lagerung von Duftstoffproben aufweist, und dass die Mittel, welche geeignet sind, eine Probe selektiv mit der Umgebungsluft in Kontakt zu bringen, eine Steuermotorvorrichtung (28) für eine Winkelverstellung der Scheibe aufweisen, um in Abhängigkeit von dem Steueralgorithmus einen der Behälter gegenüber eines Duftstoffdiffusionsfensters (20) zu positionieren.

3. Vorrichtung nach Anspruch 1 oder 2,
**dadurch gekennzeichnet,**
**dass** die Vorrichtungen (16, 22) zur Lagerung von Duftstoffproben eine Karte aufweisen, welche zerbrechliche Kammern aufweist, die mit einem Duftstoff gefüllt sind.

4. Vorrichtung nach einem der Ansprüche 1 bis 3,
**dadurch gekennzeichnet,**
**dass** sie mit mindestens einem Stecker (38, 40, 42, 43) zum Anschließen an einen Ausgang der elektronischen Einrichtung versehen ist, bei der es sich wahlweise um einen Computer, ein Multimedia-Terminal, einen digitaler Fernseher, einen digitaler Fernsehdecoder oder einen Videorecorder handeln kann.

5. Verfahren zum Diffundieren von Duftstoffen synchron mit Informationen, insbesondere Bildern, Tönen, Worten oder Ähnlichem, die einem Benutzer dargeboten werden, unter Verwendung eines elektronischen Geräts, welche mit einer Vorrichtung nach einem der Ansprüche 1 bis 4 ausgestattet ist,
**dadurch gekennzeichnet,**
**dass** es daraus besteht, dass gleichzeitig mit der Darbietung einer Folge von vorbestimmten Informationen an einen Benutzer in den Speicher der Steuereinrichtung (30) für die Vorrichtung (10) zur Diffusion von Duftstoffen ein Steueralgorithmus für die Mittel heruntergeladen wird, welche geeignet sind, eine Duftprobe selektiv in Abhängigkeit von Informationen, die dem Benutzer dargeboten werden, mit der Umgebungsluft in Kontakt zu bringen, um ihn in Hinblick auf einen späteren Ausführungsschritt zu speichern.

6. Verfahren nach Anspruch 5,
**dadurch gekennzeichnet,**
**dass** der Schritt der Ausführung des Steueralgorithmus folgende Schritte beinhaltet:
- Aktivierung der Vorrichtung (10) zur Diffusion von Duftstoffen;
- Aussendung einer Identifikationsnachricht der Mittel (16) zum Lagern von Duftstoffen, welche sich in dem Aufnahmeraum befinden;
- Aufruf eines Behälters (24) oder einer Kammer, welcher mit einem Duftstoff gefüllt ist;
- Diffusion der Duftstoffprobe, die in dem aufgerufenen Behälter (24) oder der Kammer enthalten ist;
- Anhalten der Duftstoffdiffusion nach einer vorbestimmten Zeitdauer; und
- Deaktivierung der Vorrichtung (10).

7. Verfahren nach Anspruch 6,
**dadurch gekennzeichnet,**
**dass** es nach dem Schritt des Anhaltens der Diffusion mindestens einen zweiten Schritt des Aufrufs eines anderen Behälters (24) oder einer anderen Kammer und mindestens einen zweiten Schritt der Diffusion des entsprechenden Duftstoffes beinhaltet.

8. Verfahren nach einem der Ansprüche 5 bis 7,
**dadurch gekennzeichnet,**
**dass** das elektronische Gerät aus einem Personalcomputer oder einem Multimedia-Terminal besteht, in dem, für den Zugang zu Serverzentren, über einen Zugangsanbieter als Bindeglied, ein Algorithmus zur Navigation in einem Computernetz geladen ist, und dass der Steueralgorithmus von einem Link heruntergeladen wird, der in einer Hypertext-Seite enthalten ist, welche in einem Serverzentrum gespeichert ist, mit dem der PC kommuniziert, wobei dieser Link das Herunterladen des Steueralgorithmus von einem Speicherbereich, in dem er gespeichert ist, auf den PC hervorruft.

9. Verfahren nach einem der Ansprüche 5 bis 8,
**dadurch gekennzeichnet,**
**dass** das elektronische Gerät aus einem Decoder für Digitalfernsehen besteht, wobei der Steueralgorithmus von dem Sender der digitalen Fernsehsignale übertragen und/oder aktiviert wird und dann in der Steuereinrichtung (30) der Vorrichtung (10) gespeichert wird.

## Claims

1. Device for diffusing perfumes in synchronism with data, in particular images, sounds, words or the like, displayed to a user, comprising a receptacle (14) in which are placed means (16, 22) for storing perfume samples and which is provided with means (28) for putting a sample into selective contact with the ambient air, under the control of control means (30) comprising means for storing an algorithm for controlling the operation of the said means (28) putting a sample into contact with the ambient air according to the data displayed to the user, **characterised in that** the said storage means are adapted to receive the said algorithm via downloading from electronic equipment and to store it for implementation.

2. Device according to claim 1, **characterised in that** the sample storage means (16, 22) comprise a rotary disk (22) supporting an assembly of reservoirs (24) for storing perfume samples and **in that** the means for putting a sample into contact with the ambient air include motor means (28) for controlling the angular displacement of the disk to position one of the reservoirs opposite a perfume diffusing window (20), according to the control algorithm.

3. Device according to one of claims 1 or 2, **characterised in that** the means (16, 22) for storing samples comprise a card with breakable pockets filled with perfume.

4. Device according to one of claims 1 to 3, **characterised in that** it is provided with at least one connector (38, 40, 42, 43) to connect it to an outlet of the said selected electronic equipment - a computer, a multimedia terminal, a digital television set, a digital television decoder or a video recorder.

5. Method for diffusing perfumes in synchronism with data, particularly images, sounds, words or the like, displayed to a user, at least one electronic apparatus provided with a device according to any of claims 1 to 4, **characterised in that** it consists of downloading, to the storage means (30) of the control means (10) of the perfume diffusing device, an algorithm for controlling the means which put a perfume sample into selective contact with the ambient air according to the data displayed to the user, at the same time as predetermined data is displayed to the user, and to store it for an implementation stage.

6. Method according to claim 5, **characterised in that** the stage for implementing the control algorithm comprises the following steps :
- activating the perfume diffusing device (10);
- transmitting an identification message for the sample storage means (16) arranged in the receptacle;
- addressing a reservoir (24) or pocket filled with a perfume sample;
- diffusing the perfume sample contained in the addressed reservoir (24) or pocket;
- ending perfume diffusion after a predetermined period of time; and
- deactivating the device (10).

7. Method according to claim 6, **characterised in that**, after the step for ending diffusion, it comprises at least a second step for addressing another reservoir (24) or pocket and at least a second step for diffusing the corresponding perfume sample.

8. Method according to one of claims 5 to 7, **characterised in that** the electronic apparatus consists of a micro-computer or a multimedia terminal onto which an algorithm is loaded for navigating on a data network, for access to server centres, via an access provider and **in that** the control algorithm is downloaded from a link, contained in a hypertext page stored in a server centre in communication with the micro-computer, the said link creating the downloading of the control algorithm, from a memory zone in which it is stored, to the micro-computer.

9. Method according to one of claims 5 to 8, **characterised in that** the electronic apparatus consists of a digital television decoder, the control algorithm being transmitted and/or activated by the signal transmitter of the digital television, then stored in the control means (30) of the device (10).
